Europäisches Patentamt

**European Patent Office**

Office européen des brevets

(11) Publication number: **0 093 503**
**A1**

## (12) EUROPEAN PATENT APPLICATION

(21) Application number: **83301803.9**

(22) Date of filing: **30.03.83**

(51) Int. Cl.³: **A 61 M 16/00**

(30) Priority: **07.04.82 IL 65459**

(43) Date of publication of application:
**09.11.83 Bulletin 83/45**

(84) Designated Contracting States:
**AT BE CH DE FR GB IT LI NL SE**

(71) Applicant: **SOPHISTICATED MECHANICAL EQUIPMENT LTD.**
**16/18 Bar Ilan Street**
**Tel Aviv(IL)**

(72) Inventor: **Zaferman, David**
**Rehov Meltzer 8/9**
**Rehovot(IL)**

(74) Representative: **Smith, Norman Ian et al,**
**F.J. CLEVELAND & COMPANY 40-43 Chancery Lane**
**London WC2A 1JQ(GB)**

(54) **Lung ventilator.**

(57) There is provided a ventilator which comprises inlet means connectable to a source of gas, pressure control means (20) associated with the inlet means for providing low-pressure gas to the ventilator, and means (24) for providing a constant rate gas flow. The ventilator also comprises bi-stable valve means (26) adapted to assume either a first position corresponding to the inspiration phase, or a second position corresponding to the expiration phase, pressure-sensitive means (42) for flipping the bi-stable valve means from the first to the second position and vice versa, artificial-lung means (40) for controlling the volume of gas per unit time supplied to the patient, variable flow resistances (34, 44) for adjusting the duration of the inspiration and expiration phases, and a patient connection comprising a non-rebreathing valve (46).

Fig.1.

EP 0 093 503 A1

## LUNG VENTILATOR

The present invention relates to lung ventilators and to portable, gas powered ventilators having volume and pressure controls in particular.

There are many portable or small automatic ventilators on the market. Some of these ventilators are equipped with some form of volume control (or a flow-dividing control). Others have some form of pressure control, and others combine both.

Ideally, volume of flow over the entire flow range is independent from the inspiration time. However, over the bulk of the range of flow, there is no such independence in existing ventilators.

Another desirable feature of small ventilators is resistance to vibration. Known respirators are susceptible to interruption by a sudden shock or vibration of the ventilator which can lead to grave consequences for the patient. One solution to this particular problem is provided by a ventilator which is, in principle, a pneumatic motor, and another solution by a ventilator which has no moving parts.

Known ventilators utilize an input pressure of a driving gas of not less than 2 atm. It is more desirable to utilize a lower pressure of gas.

None of the ventilators presently on the market combine all the desired properties of a portable ventilator, namely, volume and pressure control, the capability to both provide intermittent mandatory ventilation and to assist voluntary ventilation efforts of the patient, a wide flow range independent of inspiration time and resistance to vibration.

It is, therefore, an object of the present invention to provide a portable, automatic ventilator which combines all the desired properties mentioned above and whose input driving pressure is substantially lower than that of conventional ventilators.

There is thus provided in accordance with an embodiment of the present invention a ventilator comprising:

inlet means connectable to a source of gas;

pressure control means associated with said inlet means for providing low-pressure gas to said ventilator;

means for providing a constant-rate gas flow;

bi-stable valve means adapted to assume either a first position, corresponding to the inspiration phase, or a second position corresponding to the expiration phase;

pressure-sensitive means for flipping said bi-stable valve means from said first to said second positions and vice versa;

artificial-lung means for controlling the volume of gas per unit time supplied to the patient;

variable flow resistances for adjusting the duration of said inspiration and expiration phases, and

a patient connection comprising a non-rebreathing valve.

The present invention will be further understood and appreciated from the following detailed description taken in conjunction with the drawings in which:

Fig. 1 is a schematic illustration of an embodiment of the ventilator of the present invention; and

Fig. 2 is a schematic illustration of the embodiment of Fig. 1 in an alternate mode.

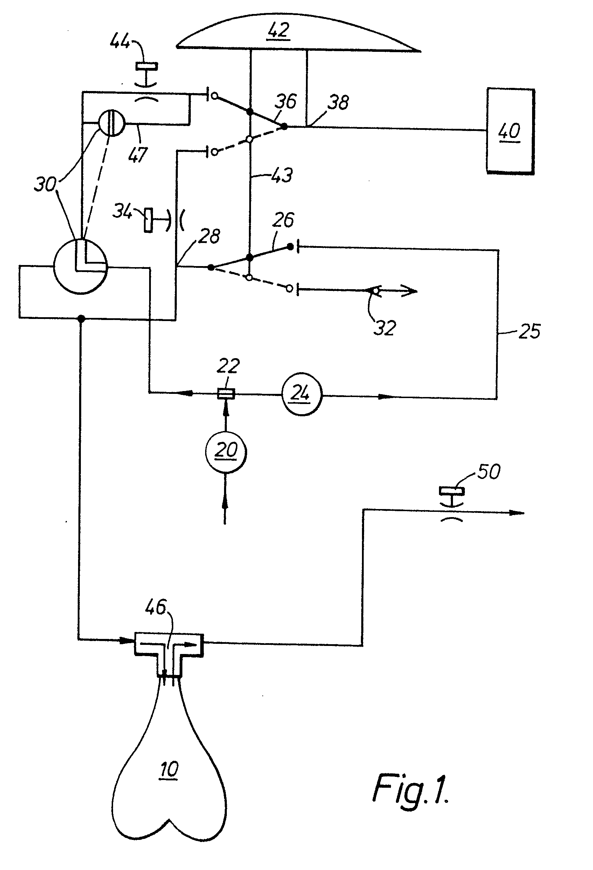

With reference to Fig. 1 there is shown a ventilator of the present invention for providing air to or assisting the voluntary ventilation of the lungs of a patient, designated 10. The ventilator comprises a source of gas (not shown) which supplies air, oxygen or a mixture thereof under pressure through pressure controller 20 which provides an output of gas at a constant low pressure. Preferably the gas leaves pressure controller 20 at a pressure of 0.5 atm.

Pressure controller 20 is connected to means for dividing the gas flow 22. Flow divider 22 acts to divide the incoming gas into two flows, each having equal, constant pressure. One flow is conducted to flow controller 24 and one flow is conducted to mode selector valve 30. Flow controller 24 acts to provide a: flow of gas at a constant rate. Flow controller 24 is coupled via gas conduit 25 to a bi-stable valve 26. Valve 26 acts selectively in one state to couple flow controller 24 to fluid conductor 28. In a second state, valve 26 acts to couple fluid conductor 28 to one-way valve 32 which permits the discharge of gas to the atmosphere.

Fluid conductor 28 is coupled via adjustable resistance 34 and, in one state, via a bi-stable valve 36 to fluid conductor 38. Resistance 34 acts to control the length of the expiration phase. Fluid conductor 38 communicates with an artificial lung 40 which controls the rhythm, or volume of gas per unit time over the inspiration-expiration cycle, of gas supplied to the patient. Artificial lung 40 is a physical model of a human lung having constant characteristics. It may comprise an elastic diaphragm or a piston system or other means which fill and empty at a constant linear functional relationship between pressure and volume.

0093503 Fluid conductor 28 is also in communication with pressure-sensitive control means 42. Control means 42 is mechanically coupled by arm 43 to bi-stable valves 26 and 36 and causes them to flip from one state to the other state responsive to the pressure therein. Control means 42 may comprise, for example, elastic diaphragm or a piston.

In a second state, valve 36 couples fluid conductor 38 via adjustable resistance 44 to mode selector valve 30. Resistance 44 acts to control the time of the inspiration phase.

Mode selector valve 30 and fluid conductor 28 are coupled to non-rebreathing valve 45. Non-rebreathing valve 46 permits gas to enter from fluid conductor 28 to lungs 10 in a uni-directional flow and, on the other side thereof, permits gas to be exhaled from lungs 10 in a uni-directional flow and discharged to the atmosphere via a controlled post-end expiratory pressure (PEEP) resistance 50.

The mode of operation illustrated in Fig. 1, the volume control mode, operates as follows. Incoming gas enters through pressure controller 20 to flow divider 22. A portion of the incoming gas is conducted through flow controller 24 via bi-stable valve 26 to fluid conductor 28 and thence through non-rebreathing valve 46 into the patient's lungs 10. This is the inspirative phase. At the same time, a constant flow of the gas from flow divider 22 passes through mode selector valve 30 via resistance 44 and bi-stable valve 36 to artificial lung 40 and control means 42. Artificial lung 40 and control means 42 expand as the volume of gas within them increases until a prescribed pressure is reached at which point control means 42 causes bi-stable valves 26 and 36 to flip to the other state (indicated by broken lines). This signals the end of the inspiration phase and the beginning of the expiration phase.

Gas from lungs 10 is expelled via PEEP resistance 50 to the atmosphere. Gas which has not yet entered non-rebreathing valve 46 returns via fluid conductor 28 and is expelled via bi-stable valve 26 and one-way valve 32 to the atmosphere. The gas from artificial lung 40 and control means 42 exits via fluid conductor 38, valve 36, through controlled resistance 34 to valve 26 and is expelled through one-way valve 32. The amount of resistance provided by resistance 34 determines the length of the expiration phase. The greater the resistance, the longer the expirative phase.

When a predetermined lower pressure is reached in artificial lung 40 and control means 42, control means 42 causes bi-stable valves 26 and 36 to flip back to the first state (as illustrated in Fig. 1) and the inspirative phase begins again.

It will be appreciated that the ventilator of the present invention can thus be utilized to assist voluntary breathing of a patient. In this case, wherein intermittent mandatory ventilation (IMV) is provided, resistance 34 is adjusted for a long expiration time from artificial lung 40 during which the patient can breathe by himself or can utilize a separate gas source, for example, using an additional IMV valve.

The ventilator of the present invention is additionally provided with an inspirational assistor to encourage voluntary breathing of the patient. If, during the expirative phase, the patient inhales, non-rebreathing valve 46 opens to permit the entry of gas into lungs 10. This lowers the gas pressure in fluid conductor 28 causing the flow through controlled resistance 34 to increase. This causes the pressure in control means 42 and artificial lung 40 to drop rapidly until the system switches to the inspiration phase (i.e. bi-stable valves 26 and 36 flip to the inspiration phase). Thus, the change of phase from expiration to inspiration occurs either when the phase is completed, as determined by the adjustment of the resistance 34, or when the patient voluntarily inhales, whichever occurs earlier.

The adjustable values of the resistances 34 and 44 are the only variables governing the work of the artificial lung which governs the rhythm of the human lungs. If the input pressure of the gas is maintained at a constant value, the timing of flow to the patient's lungs remains independent from the volume of flow throughout the entire flow range. Even if the pressure, instead of remaining at 0.5 atm oscillates between 0.45 and 0.55 atm, it has been determined experimentally that the time of the inspiration phase will change only on the order of 1%.

It will be appreciated that resistances 34 and 44 may be designed so as to maintain a constant ratio of their values. This would permit the setting of the values of both resistances with one adjustment.

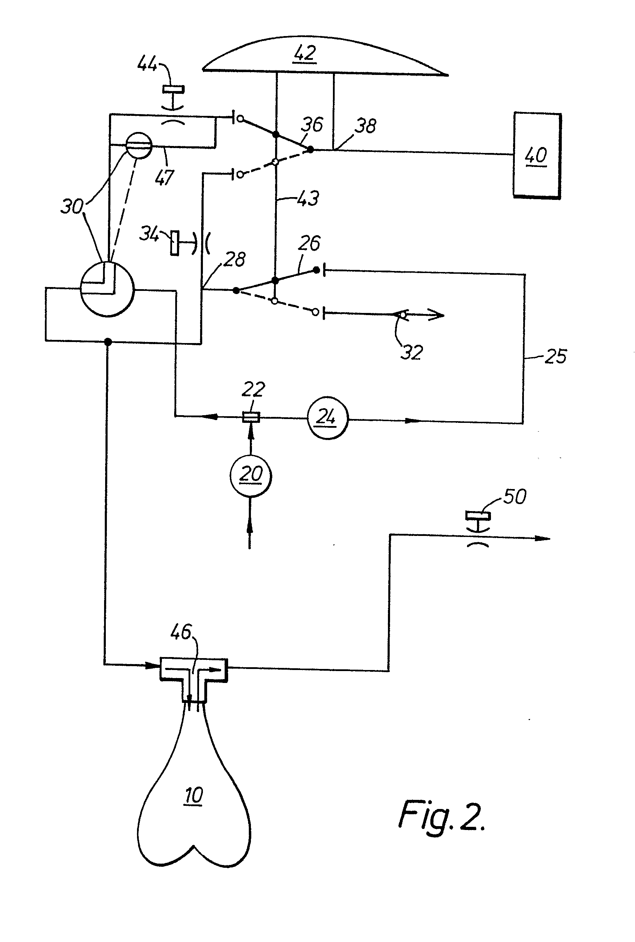

With reference to Fig. 2 there is shown the ventilator of Fig. 1 in an alternate mode, the pressure control mode. It will be noted that the ventilator has been switched to this mode by the rotation of mode selector valve 30 through 90°. In this mode, control means 42 is operated directly by the same gas pressure which acts on the patient's lungs 10 and artificial lung 40 acts only as a damper.

relatively large, for example, 200 cm$^2$. Since the weight of elements connected to artificial lung 40 and control means 42 which can be moved by vibration or sudden shock is relatively small, the change of pressure on control means 42 caused by such vibration is negligible and does not affect the operation of the ventilator. An additional advantage of using gas of low pressure is that, if the gas source is a balloon, it can be effectively utilized even when little gas remains in the canister. ¯

It should be noted that the artificial lung 40 and the control means 42 can be structurally combined in one unit.

It will be appreciated by those skilled in the art that the invention is not limited to what has been described and shown hereinabove. Rather, the scope of the invention is limited only by the claims which follow.

Gas enters pressure controller 20, as in the volume control mode, but substantially all the gas flows through flow divider 22 to flow controller 24, through gas conduit 25 and bi-stable valve 26 and into fluid conductor 28. Most of the gas is conducted to non-rebreathing valve 46 and into the lungs 10 of the patient. The rest of the flow of gas passes through mode selector valve 30 through a bypass 47 around resistance 44 and through bi-stable valve 36 to control means 42. Control means 42 reacts to a predetermined pressure to flip bi-stable valves 26 and 36. However, since the entire ventilator is at equilibrium in this mode (equal pressure), often the patient directs when to change phases. That is, there is both an inspirational and an expirational assister. Thus, if during the inspiration phase, the patient expires, the inlet to non-rebreathing valve 46 closes, there is a build-up of pressure in fluid conductor 28 which causes the pressure in control means 42 to increase rapidly so as to cause it to flip bi-stable valves 26 and 36 to the inspiration phase. Thus, the change of phase from expiration to inspiration or vice versa occurs either when the phase is completed, as determined by the adjustment of control means 42, or when the patient voluntarily inspires or expires, whichever occurs earlier.

It is a particular feature of the present invention that the ventilator is not disrupted by vibration or sudden shocks. This is due to the fact that the pressures required to cause on-off valves 26 and 36 to flip to the other phase is very low, on the order of 0.02 - 0.06 atm, for example, while the surface area of artificial lung 40 and control means 42 on which this pressure is acting is

## CLAIMS

1.   A ventilator comprising:

inlet means connectable to a source of gas;

pressure control means associated with said inlet means for providing low-pressure gas to said ventilator;

means for providing a constant-rate gas glow;

bi-stable valve means adapted to assume either a first position, corresponding to the inspiration phase, or a second position corresponding to the expiration phase;

pressure-sensitive means for flipping said bi-stable valve means from said first to said second position and vice versa;

artificial-lung means for controlling the volume of gas per unit time supplied to the patient;

variable flow resistances for adjusting the duration of said inspiration and expiration phases, and

a patient connection comprising a non-rebreathing valve.


2.   The ventilator as claimed in claim 1, further comprising a mode selector settable either to a first mode in which said bi-stable valves are flipped when pressure in patient's lungs has reached a predetermined value, or to a second mode in which said valves are flipped when the volume of gas introduced into patient's lungs has reached a predetermined value, wherein in either mode, and regardless of said predetermined values, said bi-stable valves can also be flipped by at least inspirational voluntary breathing efforts of said patient.

3.   The ventilator as claimed in claim 1, wherein said artificial-lung means comprises an elastic diaphragm.

4.   The ventilator as claimed in claim 1, wherein said artificial-lung means comprises a piston.

5.   The ventilator as claimed in claim 1, wherein said low pressure is approximately 0.5 atm.

6.   The ventilator as claimed in claim 1, further comprising a post-end expiratory pressure (PEEP) resistance downstream of said non-rebreathing valve.

7.   The ventilator as claimed in claim 1, further comprising a one-way valve which, in said exhalation phase, connects said artificial-lung means to the atmosphere.

Fig.1.

*Fig. 2.*

## EUROPEAN SEARCH REPORT

**0093503**
Application number

EP 83301803.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | US - A - 4 206 754 (COX et al.)<br>* Totality * | 1,2,3,<br>6,7 | A 61 M 16/00 |
| A | US - A - 4 265 237 (SCHWANBOM et al.)<br>* Totality * | 1,4,5 | |
| A | US - A - 4 057 059 (REID, JR. et al.)<br>* Totality; especially column 3, line 31 - column 4, line 15 * | 1,6 | |
| A | US - A - 4 232 668 (STRVPAT)<br>* Totality * | 1 | |
| A | US - A - 4 318 399 (BERNDTSON)<br>* Totality * | 1 | TECHNICAL FIELDS SEARCHED (Int. Cl. ³)<br><br>A 61 M 16/00<br>A 61 M 17/00 |
| A | US - A - 3 972 327 (ERNST et al.)<br>* Totality * | 1 | |
| A | EP - A1 - 0 026 971 (COMPAIR MAXAM)<br>* Fig. 1; abstract * | 1 | |

The present search report has been drawn up for all claims

| Place of search | Date of completion of the search | Examiner |
|---|---|---|
| VIENNA | 04-08-1983 | LUDWIG |